# EUROPÄISCHE PATENTANMELDUNG

(11) **EP 2 997 896 A1**
(43) Veröffentlichungstag der Anmeldung: **23.03.2016**
(21) Anmeldenummer: 15183257.3
(22) Anmeldetag: 12.01.2010
(51) Int. Cl.: A61B 5/145, G01N 21/77, A61B 5/155, A61B 5/1455

(54) **VORRICHTUNG ZUR MESSUNG ZUMINDEST EINES PARAMETERS EINER ARTERIELLEN BLUTPROBE**

(30) Priorität: 13.01.2009 AT 462009
(62) Teilanmeldung aus: 10700321.2
(71) Anmelder: Smart Medical Solutions GmbH, 8047 Graz (AT)
(72) Erfinder: KÖHLER, Hans, 8046 Graz (AT)
(74) Vertreter: Babeluk, Michael

(57) **Zusammenfassung**

Eine Vorrichtung zur Messung zumindest eines Parameters einer arteriellen Blutprobe weist einen Durchflusssensor (2) mit standardisierten Anschlusselementen (3, 4) auf, die mit der Einlass- und der Auslassseite (5, 6) der Messzelle (7) des Durchflusssensors (2) in Verbindung stehen, wobei die Messzelle (7) zumindest ein mit der Blutprobe in Kontakt bringbares optochemisches Sensorelement (8) aufweist, und wobei der Durchflusssensor (2) mit dem einlassseitigen Anschlusselement (3) an den standardisierten Anschluss (14) eines arteriellen Katheters (1) und mit dem auslassseitigen Anschluss (4) an den standardisierten Anschluss (15) eines arteriellen Infusionsbestecks (16) anschließbar ist. Erfindungsgemäß ist ein auf den Durchflusssensor (2) abnehmbar, aufsteckbarer Konnektor (9) mit zumindest einer Lichtquelle (10) zur Anregung des optochemischen Sensorselementes (8) und zumindest einem Fotodetektor (11) zur Aufnahme der Messstrahlung des optochemischen Sensorelementes (8) ausgestattet. Ein elektronisches Modul (12), welches eine elektrische Verbindungsleistung (13) zum Konnektor (9) aufweist, kontaktiert die zumindest eine Lichtquelle (10) und den zumindest einen Fotodetektor (11). Weiters ist in der Messzelle (7) des Durchflusssensors (2) eine vorzugsweise optische Messeinrichtung angeordnet, die bei Kontakt mit der Blutprobe ein Startsignal zur Aktivierung der Parametermessung abgibt.

## Beschreibung

Die Erfindung betrifft ein Verfahren und eine Vorrichtung zur Messung zumindest eines Parameters einer arteriellen Blutprobe.

Um beispielsweise auf Intensivstationen bei bereits liegendem, arteriellem Zugang eine Blutprobe abzunehmen, wird mit einem Aspirationskolben des Infusionsbestecks Blut manuell aspiriert und mit Hilfe einer Wegwerfspritze über das Septum des Infusionsbestecks entnommen. Die Blutprobe wird anschließend mittels externem Blutgasanalysator analysiert bzw. nach der Transferierung in Abnahmeröhrchen zur Analyse in ein Labor verschickt. Bei der Entnahme über das Septum besteht eine gewisse Infektions- und Verletzungsgefahr für den Patienten und das Personal, wobei beim Transport zum externen Blutgasanalysator bzw. beim Verschicken zur Laboranalyse Fehler auftreten können, z.B. durch falsch etikettierte bzw. verwechselte Blutproben.

In diesem Zusammenhang ist es auch bekannt geworden, bestimmte Blutparameter direkt am Patienten zu vermessen. So ist aus der US 4,989,606 A ein intravaskuläres Blutgasmesssystem bekannt, bei welchem eine mit dem vaskulären System des Patienten kommunizierende Schlauchleitung direkt an eine Sensoreinheit angeschlossen ist. Die Sensoreinheit weist einen Durchflusssensor mit fluoreszenzoptischen Sensoren auf, beispielsweise zur Messung der O₂- und der CO₂-Konzentration, sowie des pH-Wertes des Blutes. Ausgangsseitig ist an dem Durchflusssensor eine Schlauchleitung fixiert, die mit einem Druckbeutel in Verbindung steht, der eine heparinisierte Salzlösung enthält. In der Schlauchleitung zum Druckbehälter ist ein einstellbares Tropfventil und ein manuell betätigbares Spülventil angeordnet.

Der Durchflusssensor der US 4,989,606 A ist an einer Übertragungseinheit befestigt, welche eine Thermostatisiereinrichtung für die Blutprobe, sowie mehrere Lichtleiter enthält, mit welchen die optischen Sensoren mit Anregungsstrahlung versorgt werden und die Messstrahlung abgeführt wird. Die am Arm des Patienten fixierte Sensoreinheit ist relativ großvolumig und schwer und wird daher im Einsatz meist als störend empfunden. Weiters ist das Messsystem nicht in herkömmliche Infusionsbestecke integrierbar.

Die Übertragung der Anregungsstrahlung und der Messstrahlung zwischen der Übertragungseinheit und einem Auswerteinstrument der US 4,989,606 A erfolgt über Lichtleiter, wodurch die Bewegungsfreiheit des Patienten eingeschränkt werden kann.

Aus der EP 0 994 669 B1 ist ein System zum Messen von Blutparametern bekannt geworden, das bei chirurgischen Eingriffen im extrakorporalen Kreislauf (Herz-Lungen-Maschine) eingesetzt werden kann. Die Vorrichtung weist eine Durchflusskassette mit einer Kammer zur Aufnahme der Blutprobe auf, wobei in der Kassette mindestens ein optischer Sensor angeordnet ist. Weiters ist eine Vorrichtung vorgesehen, mit der die Kassette lösbar verbunden werden kann, wobei die Vorrichtung eine Lichtquelle umfasst, mit der der Sensor beaufschlagt wird, weiters einen Lichtdetektor zum Detektieren von Licht, das vom Sensor ausgeht, und mindestens einen Signalumsetzer, der mit dem Lichtdetektor verbunden ist. Der Signalumsetzer stellt ein digitales Ausgangssignal bereit, das sich als Antwort auf die Lichtmenge ändert, die von mindestens einem Lichtdetektor detektiert wird. Hier gelten die bereits oben zur US 4,989,606 angeführten Nachteile, wonach die Vorrichtung großvolumig ausgeführt und nicht in herkömmliche Infusionsbestecke integrierbar ist.

Aus der US 4,830,013 A ein intravaskuläres Messsystem für Blutparameter bekannt, welches eine Kathetereinheit aufweist, in deren distalen Endbereich optische Sensoren zur Messung der Blutgase (O₂-Konzentration, CO₂-Konzentration und pH-Wert) vorgesehen sind, die jeweils an der Spitze eines Lichtleiters angeordnet sind.

Schließlich ist aus der WO 99/02084 A1 eine Vorrichtung zur Messung zumindest eines Parameters einer arteriellen Blutprobe bekannt, die eine auswechselbare Sensor-Cartridge mit standardisierten Anschlusselementen aufweist, die mit der Einlass- und der Auslassseite einer Messzelle in Verbindung stehen, wobei die Messzelle zumindest ein mit der Blutprobe in Kontakt bringbares optochemisches Sensorelement aufweist, das mit seiner Messspitze in die Messzelle ragt. Weiters ist es aus der WO 99/02084 A1 bekannt, die Sensor-Cartridge über seine standardisierten Anschlüsse in das arterielle Infusionsbesteck für einen Patienten zu integrieren. Die Messzelle weist eine Temperaturmesseinrichtung und eine Heizeinrichtung auf, mit welcher die Probentemperatur in gewünschter Weise justiert werden kann.

Aufgabe der Erfindung ist es, eine Vorrichtung zur Messung zumindest eines Parameters einer arteriellen Blutprobe vorzuschlagen, die möglichst kleinvolumig und leicht handhabbar in herkömmliche arterielle Zugangssysteme einsetzbar ist.

Diese Aufgabe wird erfindungsgemäß durch eine Vorrichtung mit einen Durchflusssensor mit standardisierten Anschlusselementen, die mit der Einlass- und der Auslassseite der Messzelle des Durchflusssensors in Verbindung stehen, wobei die Messzelle zumindest ein mit der Blutprobe in Kontakt bringbares optochemisches Sensorelement aufweist, mit folgenden Komponenten gelöst:
- einen auf den Durchflusssensor abnehmbar, aufsteckbaren Konnektor mit zumindest einer Lichtquelle zur Anregung des optochemischen Sensorelementes und zumindest einem Fotodetektor zur Aufnahme der Messstrahlung des optochemischen Sensorelementes,
- ein elektronisches Modul, welches eine elektrische Verbindungsleitung zum Konnektor aufweist und die zumindest eine Lichtquelle und den zumindest einen Fotodetektor kontaktiert, und
- eine in der Messzelle des Durchflusssensors angeordnete, vorzugsweise optische Messeinrichtung, die bei Kontakt mit der Blutprobe ein Startsignal zur Aktivierung der Parametermessung abgibt.

Die erfindungsgemäße Vorrichtung lässt sich optimal in den klinischen Arbeitsablauf einfügen, wobei zum Applizieren der Messvorrichtung lediglich die standardisierte Verbindung zwischen dem arteriellen Katheter und dem arteriellen Infusionsbesteck aufgetrennt und das erfindungsgemäße System eingesteckt werden muss. Das elektronische Modul, welches die Lichtquellen und die Fotodetektoren im Konnektor versorgt, ist über eine flexible elektrische Verbindungsleitung mit einer Auswerte- und Anzeigeeinheit verbunden, bzw. kann auch über eine kabellose Funkverbindung verfügen, so dass ohne weitere Kabelverbindung ein Kontakt zur Auswerte- und Anzeigeeinheit hergestellt werden kann. Der Durchflusssensor samt Konnektor kann am Arm des Patienten fixiert werden, wobei das elektronische Modul ebenfalls am Arm fixiert oder in der Auswerte- und Anzeigeeinheit integriert sein kann.

Gemäß einer vorteilhaften Ausführungsvariante der Erfindung kann in der Messzelle des Durchflusssensors eine vorzugsweise optische Messeinrichtung angeordnet sein, die bei Kontakt mit der Blutprobe ein Startsignal zur Aktivierung der Parametermessung abgibt.

Die Erfindung wird im Folgenden anhand von Zeichnungen näher erläutert. Es zeigen:
- Fig. 1: ein arterielles Zugangssystem samt Katheter und Infusionsbesteck gemäß Stand der Technik;
- Fig. 2: eine Ausführungsvariante der erfindungsgemäßen Vorrichtung zur Messung zumindest eines Parameters einer arteriellen Blutprobe, integriert in ein arterielles Zugangssystem gemäß Fig. 1;
- Fig. 3: und Fig. 4 unterschiedliche Ausführungsdetails der erfindungsgemäßen Vorrichtung gemäß Fig. 2; sowie
- Fig. 5: eine bevorzugte Ausführungsvariante der erfindungsgemäßen Vorrichtung gemäß Fig. 2.

Ein arterieller Zugang gemäß Stand der Technik, mit welchem eine Blutprobe zur Blutanalyse entnommen werden kann, weist einen arteriellen Katheter 1 sowie ein direkt aufsteckbares arterielles Infusionsbesteck 16 auf. Ausgangsseitig des Infusionsbestecks 16 befindet sich ein nur schematisch angedeuteter Druckbeutel 20, der beispielsweise eine heparinisierte Spüllösung enthält.

Für eine durchzuführende Blutanalyse muss eine Blutprobe entnommen werden, wozu folgende Arbeitsschritte notwendig sind:
1. Das Sperrventil 21 zwischen Druckbeutel 20 und dem Aspirationskolben 22 wird geschlossen.
2. Eine Blutprobe wird manuell über den Aspirationskolben 22 des Infusionsbestecks 16 aspiriert.
3. Das Sperrventil 23 zwischen Aspirationskolben 22 und dem Septum 24 zur Blutentnahme wird geschlossen.
4. Eine arterielle Blutprobe wird mittels einer Wegwerfspritze (nicht dargestellt) über das Septum 24 entnommen (Nachteile: Infektionsgefahr für Patienten und Personal, Verletzungsgefahr).
5. Die Blutprobe wird mittels eines externen Analysators vermessen bzw. in Aufnahmeröhrchen transferiert und zur Analyse in ein Labor verschickt. (Nachteile: erhöhter Manipulationsaufwand, Gefahr der Verunreinigung)
6. Das Sperrventil 23 wird geöffnet und das aspirierte Blut mittels Aspirationskolben 22 in die Arterie reinfundiert.
7. Das Septum 24 wird gereinigt.
8. Das Sperrventile 21 wird geöffnet und die arterielle Leitung über das Spülventil 26 gereinigt.

Das manuell einstellbare Tropfventil des Infusionsbestecks ist mit 25 bezeichnet. Der arterielle Katheter 1 sowie das Infusionsbesteck 16 weisen standardisierte Anschlüsse 14, 15, beispielsweise Luer-Adapter auf, mit welchen die beiden Komponenten 1 und 16 in bekannter Weise flüssigkeits- und gasdicht verbunden werden.

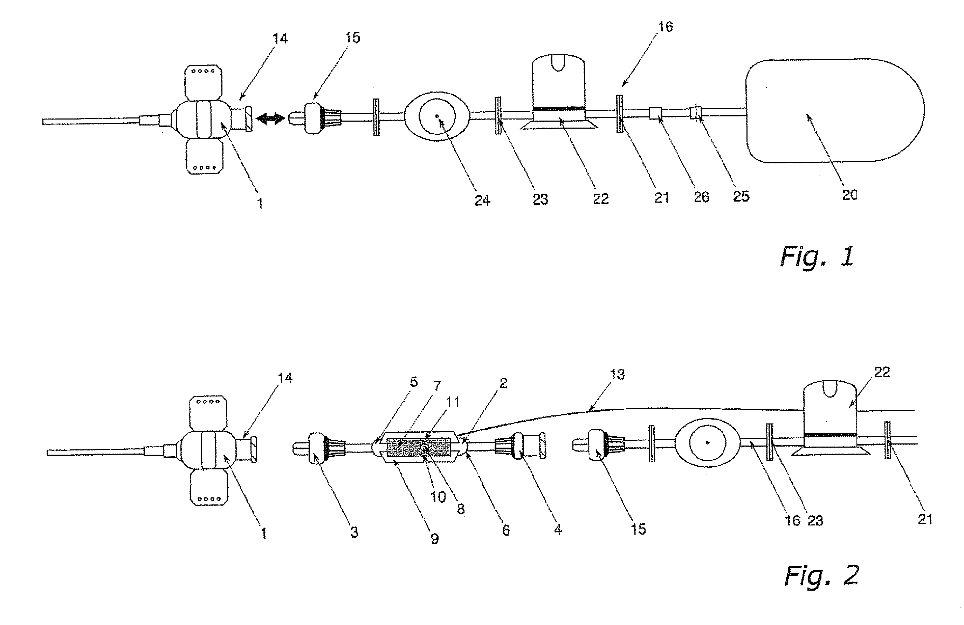

Das erfindungsgemäße Messsystem gemäß Fig. 2 weist im Gegensatz dazu wesentliche Vorteile auf, da hier die Messung der gewünschten Blutparameter direkt am Patienten mit einem Durchflusssensor 2 erfolgt, der zwischen die handelsüblichen Komponenten eines arteriellen Zugangs, nämlich einem arteriellen Katheter 1 und einem arteriellen Infusionsbesteck 16 eingefügt wird.

Der Durchflusssensor 2 der erfindungsgemäßen Vorrichtung weist zu diesem Zweck standardisierte Anschlusselemente 3 und 4 auf, beispielsweise Luer-Adapter, die mit der Einlass- und der Auslassseite 5, 6 der Messzelle 7 des Durchflusssensors 2 in Verbindung stehen. In der Messzelle 7 sind die mit der Blutprobe in Kontakt bringbaren optochemischen Sensorelemente 8 angeordnet, wobei in Fig. 2 schematisch lediglich ein derartiges Sensorelement dargestellt ist.

Weiters weist die Vorrichtung einen auf den Durchflusssensor 2 abnehmbar aufsteckbaren Konnektor 9 auf, der zumindest mit einer Lichtquelle 10 zur Anregung des optochemischen Sensorelementes 8 und einem Fotodetektor 11 zur Aufnahme der vom Sensorelement 8 emittierten Messstrahlung ausgestattet ist. Das Messsystem kann daher sehr kostengünstig betrieben werden, da der Durchflusssensor für einen neuen Messeinsatz der Vorrichtung einfach ausgewechselt werden kann.

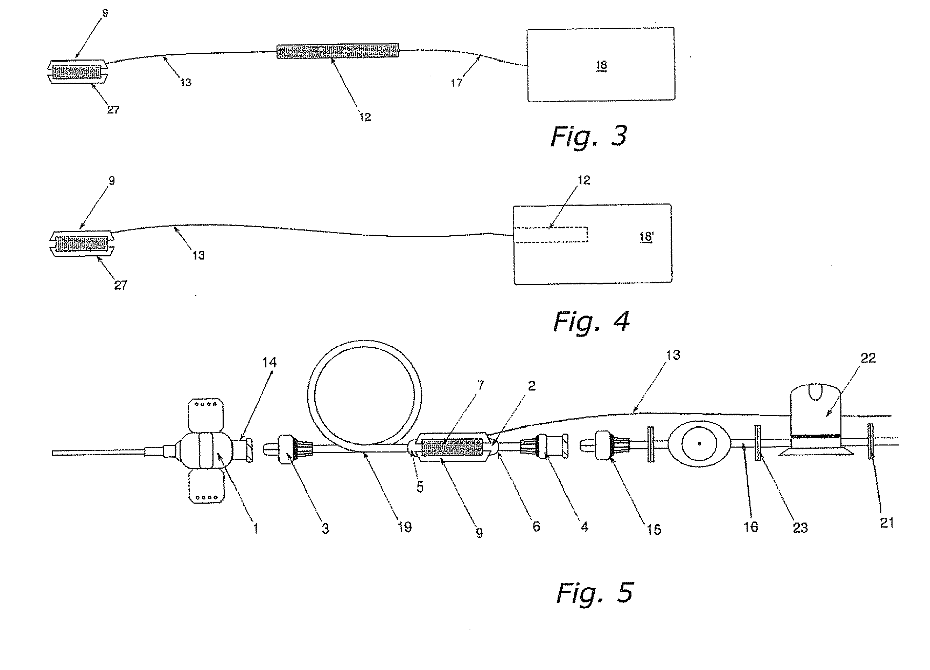

Wie weiters in Fig. 3 dargestellt, ist der Konnektor 9 über eine elektrische Verbindungsleitung 13 mit einem elektronischen Modul 12 verbunden, welches zur Versorgung der Lichtquelle 10 (z.B. LED) und zur Weiterleitung der Signale des Fotodetektors 11 (z.B. Fotodiode) dient. Der Durchflusssensor 2 ist mit dem einlassseitigen Anschlusselement 3, an den standardisierten Anschluss 14 des arteriellen Katheters 1 und mit dem auslassseitigen Anschluss 4 mit den standardisierten Anschluss 15 des arteriellen Infusionsbestecks 16 angeschlossen. Das gesamte Messsystem kann somit auf einfache Weise durch Auftrennen der Luer-Verbindung in ein handelsübliches arterielles System gemäß Fig. 1 eingefügt werden.

Das elektronische Modul 12, das zur elektrischen Kontaktierung der Lichtquellen 10 und der Fotodetektoren 11 im Konnektor 9 dient, ist über eine elektrische Verbindungsleitung 17 oder eine kabellose Funkverbindung mit einer Auswerte- und Anzeigeeinheit 18 verbunden. Wie in Fig. 4 schematisch dargestellt, kann das elektronische Modul 12 auch direkt in eine Auswerte- und Anzeigeeinheit 18' integriert sein. Der Konnektor 9 weist eine den Durchflusssensor 2 mechanisch stabil umfassende Klemme 27 auf, durch die eine lagerichtige Fixierung der optischen Elemente erfolgt.

Bei der in Fig. 5 dargestellten Ausführungsvariante der erfindungsgemäßen Vorrichtung ist zur mechanischen Entlastung des arteriellen Katheters 1 zwischen dem einlassseitigen Anschlusselement 3 des Durchflusssensors 2 und der eigentlichen Messzelle 7 ein flexibles Schlauchstück 19 vorgesehen. Eine derartige flexible Entlastung kann auch zwischen dem auslassseitigen Anschlusselement 4 und der Messzelle 7 vorgesehen sein.

Gemäß einer Ausführungsvariante der Erfindung kann in der Messzelle 7 des Durchflusssensors 2 eine vorzugsweise optische Messeinrichtung angeordnet sein, die bei Kontakt mit der Blutprobe ein Startsignal zur Aktivierung der Parametermessung abgibt. Mit dieser Einrichtung kann auch unbeabsichtigt in das Entnahmesystem austretendes Blut rasch detektiert und von der Auswerte- und Anzeigeeinheit 18, 18' ein entsprechendes optisches oder akustisches Warnsignal sowie ggf. eine Spülung des Katheters aktiviert werden.

Zur Blutanalyse mit der erfindungsgemäßen Vorrichtung sind lediglich folgende Arbeitsschritte auszuführen:
1. Schließen des Sperrventils 21 zwischen Druckbeutel 20 und Aspirationskolben 22;
2. manuelles Aspirieren einer Blutprobe über den Aspirationskolben 22 des arteriellen Infusionsbesteckes 16. Anschließend kann die Messung durch eine optische Bluterkennungseinrichtung automatisch aktiviert werden;
3. Abwarten eines Bestätigungssignals (akustisch oder optisch) der Auswert- und Anzeigeeinheit 18 bzw. 18' über die erfolgt Messung;
4. Reinfundieren der Blutprobe mittels Aspirationskolben 22;
5. Spülen der arteriellen Leitungen mit dem Spülventil 26

Es ist auch möglich über ein automatisiertes System in vorgegebenen Zeitabständen Messungen vorzusehen, wobei die Aspiration der Blutprobe in die Messzelle 7 des Durchflusssensors 2 mit einer (hier nicht dargestellten) automatisch betätigbaren Kolben- oder Schlauchpumpe erfolgt.

Erfindungsgemäß kann in der Messzelle 7 des Durchflusssensors 2 zusätzlich ein Sensor zur Temperaturmessung angeordnet sein. Bevorzugt wird die Messung erst dann gestartet, wenn die Blutprobe zu einem Stillstand gekommen ist. Der automatische Start der Messung kann somit mit einer Temperaturmessung gekoppelt werden. Die Fließgeschwindigkeit bzw. der Stillstand der Blutprobe kann beispielsweise durch den gemessenen Temperaturgradienten bestimmt werden. Das Prinzip: Solange das aspirierte Blut in Bewegung ist, wird die Temperatur ansteigen. Sobald das Blut in der Messzelle steht wird die Temperatur beginnen abzufallen. So kann der optimale Startzeitpunkt der Messung besser bestimmt und kontrolliert werden, was die Messgenauigkeit verbessert.

Bevorzugt weist die Messzelle 7 des Durchflusssensors 2 zumindest einen Sensor zur Messung eines Parameters aus der Gruppe O₂, CO₂, pH, Natrium, Kalium, Glukose, Laktat und Temperatur auf.

Die wesentlichen Vorteile des erfindungsgemäßen Messsystems bestehen somit darin:
- Das Messsystem kann auf einfache Weise in ein handelsübliches System aus einem arteriellen Katheter und einem arteriellen Infusionsbesteck mittels standardisierten Anschlüssen (z.B. Luer) eingefügt werden.
- Die erfindungsgemäße Vorrichtung kann somit mit jedem herkömmlichen Blutentnahmesystem verwendet werden.
- Der arterielle Katheter kann für sämtliche vorgesehene Anwendungen weiter in Benutzung bleiben.
- Es besteht keine Infektionsgefahr für Patienten und Personal.
- Kein Blutverbrauch durch die Messung.
- Es besteht keine Verwechslungsgefahr bei der Analyse einer Blutprobe.

## Patentansprüche

1. Vorrichtung zur Messung zumindest eines Parameters einer arteriellen Blutprobe, mit einem Durchflusssensor (2) mit standardisierten Anschlusselementen (3, 4), die mit der Einlass- und der Auslassseite (5, 6) der Messzelle (7) des Durchflusssensors (2) in Verbindung stehen, wobei die Messzelle (7) zumindest ein mit der Blutprobe in Kontakt bringbares optochemisches Sensorelement (8) aufweist und wobei der Durchflusssensor (2) mit dem einlassseitigen Anschlusselement (3) an den standardisierten Anschluss (14) eines arteriellen Katheters (1) und mit dem auslassseitigen Anschluss (4) an den standardisierten Anschluss (15) eines arteriellen Infusionsbestecks (16) anschließbar ist,
**gekennzeichnet durch**:
- einen auf den Durchflusssensor (2) abnehmbar, aufsteckbaren Konnektor (9) mit zumindest einer Lichtquelle (10) zur Anregung des optochemischen Sensorelementes (8) und zumindest einem Fotodetektor (11) zur Aufnahme der Messstrahlung des optochemischen Sensorelementes (8),
- ein elektronisches Modul (12), welches eine elektrische Verbindungsleitung (13) zum Konnektor (9) aufweist und die zumindest eine Lichtquelle (10) und den zumindest einen Fotodetektor (11) kontaktiert, und
- eine in der Messzelle (7) des Durchflusssensors (2) angeordnete, vorzugsweise optische Messeinrichtung, die bei Kontakt mit der Blutprobe ein Startsignal zur Aktivierung der Parametermessung abgibt.

2. Vorrichtung nach Anspruch 1, **dadurch gekennzeichnet, dass** in der Messzelle (7) des Durchflusssensors (2) zusätzlich ein Sensor zur Temperaturmessung angeordnet ist.

3. Vorrichtung nach Anspruch 1 oder 2, **dadurch gekennzeichnet, dass** das elektronische Modul (12) eine elektrische Verbindungsleitung (17) oder eine kabellose Funkverbindung zu einer Auswerte- und Anzeigeeinheit (18) aufweist.

4. Vorrichtung nach Anspruch 1 oder 2, **dadurch gekennzeichnet, dass** das elektronische Modul (12) in eine Auswerte- und Anzeigeeinheit (18') integriert ist.

5. Vorrichtung nach einem der Ansprüche 1 bis 4, **dadurch gekennzeichnet, dass** die Messzelle (7) des Durchflusssensors (2) zumindest einen Sensor zur Messung eines Parameters aus der Gruppe O₂, CO₂, pH, Natrium, Kalium, Glukose, Laktat und Temperatur aufweist.

6. Vorrichtung nach einem der Ansprüche 1 bis 5, **dadurch gekennzeichnet, dass** als Lichtquelle (10) eine LED und als Fotodetektor (11) eine Fotodiode vorgesehen ist.

7. Verfahren zur Messung zumindest eines Parameters einer arteriellen Blutprobe, wobei ein Durchflusssensor (2), der in seiner Messzelle (7) zumindest ein optochemisches Sensorelement (8) aufweist, mit seinen standardisierten Anschlusselementen (3, 4) in ein arterielles Infusionsbesteck (16) eingesteckt wird, indem der Durchflusssensor (2) mit dem einlassseitigen Anschlusselement (3) an den standardisierten Anschluss (14) eines arteriellen Katheters (1) und mit dem auslassseitigen Anschluss (4) an den standardisierten Anschluss (15) des arteriellen Infusionsbestecks (16) angeschlossen wird,
**gekennzeichnet durch**:
- Aufstecken eines Konnektors (9) auf den Durchflusssensor (2) welcher Konnektor (9) zumindest einer Lichtquelle (10) zur Anregung des optochemischen Sensorelementes (8) und zumindest einem Fotodetektor (11) zur Aufnahme der Messstrahlung des optochemischen Sensorelementes (8) aufweist, sowie
- Aktivieren eines Startsignals zur Parametermessung **durch** eine in der Messzelle (7) des Durchflusssensors (2) angeordnete, vorzugsweise optische Messeinrichtung bei Kontakt mit der Blutprobe.

8. Verfahren nach Anspruch 7, **dadurch gekennzeichnet, dass** mit der vorzugsweise optischen Messeinrichtung unbeabsichtigt in das Entnahmesystem austretendes Blut detektiert und ein entsprechendes optisches oder akustisches Warnsignal aktiviert wird.

9. Verfahren nach Anspruch 8, **dadurch gekennzeichnet, dass** eine Spülung des arteriellen Katheters (1) aktiviert wird.

10. Verfahren nach einem der Ansprüche 7 bis 9, **dadurch gekennzeichnet, dass** mit Hilfe eines zusätzlichen Sensors zur Temperaturmessung in der Messzelle (7) des Durchflusssensors (2) ein Temperaturgradient gemessen und die Fließgeschwindigkeit oder der Stillstand der Blutprobe bestimmt wird.

11. Verfahren nach Anspruch 10, **dadurch gekennzeichnet, dass** der Start der Parametermessung mit der Temperaturmessung gekoppelt wird und die Messung bevorzugt erst dann gestartet wird, wenn die Blutprobe zu einem Stillstand gekommen ist.
